# EUROPEAN PATENT SPECIFICATION

(11) **EP 3 695 201 B1**
(45) Date of publication and mention of the grant of the patent: **02.12.2026**
(21) Application number: 18793345.2
(22) Date of filing: 09.10.2018
(51) Int. Cl.: G01N 1/31, G01N 35/00, G01N 1/36

(54) **TISSUE CASSETTE READER**
GEWEBEKASSETTENLESEGERÄT
LECTEUR DE CASSETTE D'INCLUSION

(30) Priority: 09.10.2017 US 201762570077 P; 08.10.2018 US 201816154634
(43) Date of publication of application: 19.08.2020
(73) Proprietor: Sakura Finetek U.S.A., Inc., Torrance, CA 90501 (US)
(72) Inventor: VON BUEREN, Erico, Torrance California 90501 (US); GREENLEE, Joshua, Torrance California 90501 (US)
(74) Representative: Studio Torta S.p.A.
(86) International application number: PCT/US2018/055037
(87) International publication number: WO 2019/074941

(56) References cited:
- EP-A1- 3 200 118
- WO-A1-2014/130592
- US-A1- 2010 086 964
- US-A1- 2013 065 797
- US-A1- 2014 273 085
- IDMAG ROMANIA: "Scandit Barcode Scanner SDK 5 2 Includes MatrixScan Premium", YOUTUBE, 6 July 2017 (2017-07-06), XP055544932, Retrieved from the Internet <URL:https://www.youtube.com/watch?v=xe0NuLFC9_o> [retrieved on 20190118]

## Description

### CROSS-REFERENCE TO RELATED APPLICATIONS

The present Application is a continuation of United States Patent Application No. 16/154,634 entitled "Tissue Cassette Reader," filed October 8, 2018, which claims the benefit of United States Provisional Patent Application No. 62/570,077 entitled "Tissue Cassette Reader," filed October 9, 2017.

### TECHNICAL FIELD

Tissue processing and carrier identification.

### BACKGROUND

Tissues from the body taken for diagnosis of disease processes are often processed in the histology laboratory to produce paraffin blocks embedding them to then cut thin tissue sections which can be mounted on slides, stained and viewed under a microscope by a pathologist for analysis. These pre-analytical processes generally include, in order, gross examination, fixation, dehydration, clearing, paraffin infiltration and embedding. The procedure is used for processing tissues including biopsies, larger specimens removed at surgery, or tissues from autopsy.

Gross examination generally consists of describing the macroscopic specimen and placing all or selected parts of it into a sample carrier such as a small plastic cassette which holds the tissue while it is being processed to a paraffin block. Initially, the cassettes are placed into a fixative.

Following gross examination, the fixation of the tissue continues. A purpose of fixation is to preserve tissues permanently in as life-like a state as possible by altering structures of proteins such that degradation by autolysis does not occur. Once the tissue has been fixed or fixated, the tissue needs to be processed into a form in which it can be made into thin sections for microscopic examination. The usual way this is done is with paraffin.
Embedding tissue in paraffin provides a solid support matrix for the tissue allowing it be sectioned at a thickness on the order of 1 to 20 microns. Getting fixed tissue into paraffin for sectioning is called tissue processing with the main steps in this process being dehydration, clearing, infiltration, which then is followed by embedding.

Tissues fixed in aqueous solutions cannot be directly infiltrated with paraffin. First, the water from the tissues must be removed by dehydration. This may be done with a series of alcohols at different concentrations (e.g., 70 percent to 95 percent to 100 percent). Alternatively, the dehydration is done with a mixture of formalin and alcohol. Other dehydrants can also be used such as acetone or mixtures of different solvents.

Following dehydration, the tissue is cleared. "Clearing" consists of removal of the dehydrant and some of the lipids with a substance that will be miscible with the embedding medium (e.g., paraffin). The most common clearing agent is xylene.

Once cleared, the tissue is infiltrated with an embedding agent such as paraffin. Finally, the tissue in a cassette or removed from its cassette is placed into molten paraffin and then the paraffin is cooled to form a solidified block embedding or encapsulating the tissue so that it can be sectioned. Alternatively, the tissue can be processed in a sectionable cassette, embedded in paraffin along with the cassette and sectioned. Once the tissue has been embedded in a solid paraffin block, the tissue can be cut into sections that can be placed on one or multiple slides. This is done with a microtome. Once sections are cut, they are floated on a warm water bath that helps remove any wrinkles. The tissue sections in paraffin are then picked up from the water bath and placed on a glass microscope slide.

A sample carrier such as a cassette may be marked with identification and/or process information. The introduction of barcodes has made it possible to machine read barcodes printed on a sample carrier and to track the sample carrier during histological sample processing, embedding, sectioning and any verifications steps from creation to archiving.

WO 2014/130592 A1 describes a tissue cassette magazine and removing a tissue cassette from the magazine before imaging a single cassette. US 2010/086964 A1 describes an apparatus and a method for tagging a slide to a cassette. *"Scandit Barcode Scanner SDK 5 2 Includes MatrixScan Premium"* by Idmag Romania discloses an analyzer unit including an image pick up including a CCD sensor to provide an image of operations in an analyzer unit that are displayed on the unit. US 2014/273085 A1 discloses a processor for working on histological samples, the processor has an inlet for the insertion of a fixing container, particularly a closed fixing container, with the samples located in the latter. EP 3 200 118 A1 discloses a sample traceability device and method for medical research and/or diagnosis. US 2013/065797 A1 discloses a management system for automated sample processing where a sensor is moveable, referencing a camera mounted on a robot arm.

### BRIEF DESCRIPTION OF THE DRAWINGS

Embodiments are illustrated by way of example and not by way of limitation in the figures of the accompanying drawings in which like references indicate similar elements. It should be noted that references to "an" or "one" embodiment in this disclosure are not necessarily to the same embodiment, and such references mean at least one.
**Figure 1** shows an illustrative process flow of preparing a tissue taken from a subject for tissue processing.
**Figure 2** shows a top, side perspective view of an embodiment of an electronic device that can sense (read), store and output identification information such as a barcode on a sample carrier.
**Figure 3** shows the device of **Figure 1** where a lid of the device is rotated over a base of the device.
**Figure 4** shows a top, side perspective view of another embodiment of an electronic device that can sense (scan), store and output identification information such as a barcode on a sample carrier.
**Figure 5** shows the device of **Figure 4** showing the scanning of four identification information on cassettes in four magazines in a container placed in a recess in a base of the device.

### DETAILED DESCRIPTION

The invention is set out in the attached set of claims. An object of the invention is to provide an automated device or apparatus operable to obtain information from a sample carrier such as an identifier on a sample carrier such as a tissue cassette is disclosed. In one embodiment, the device is operable to sense identification information (an identifier) such as a barcode on a display area of a single cassette while the cassette is present with other cassettes in an assembly such as a magazine or basket. In another embodiment, the device or apparatus is operable to capture an image (e.g., a two- or three-dimensional image) of identifiers on multiple cassettes in an assembly. Each identifier whether sensed individually or captured in an image of identifiers of multiple cassettes is then read and optionally stored. The read and optionally stored information regarding an identifier may be used for quality control, routing and tracking of cassettes in a laboratory (e.g., a histopathology laboratory).

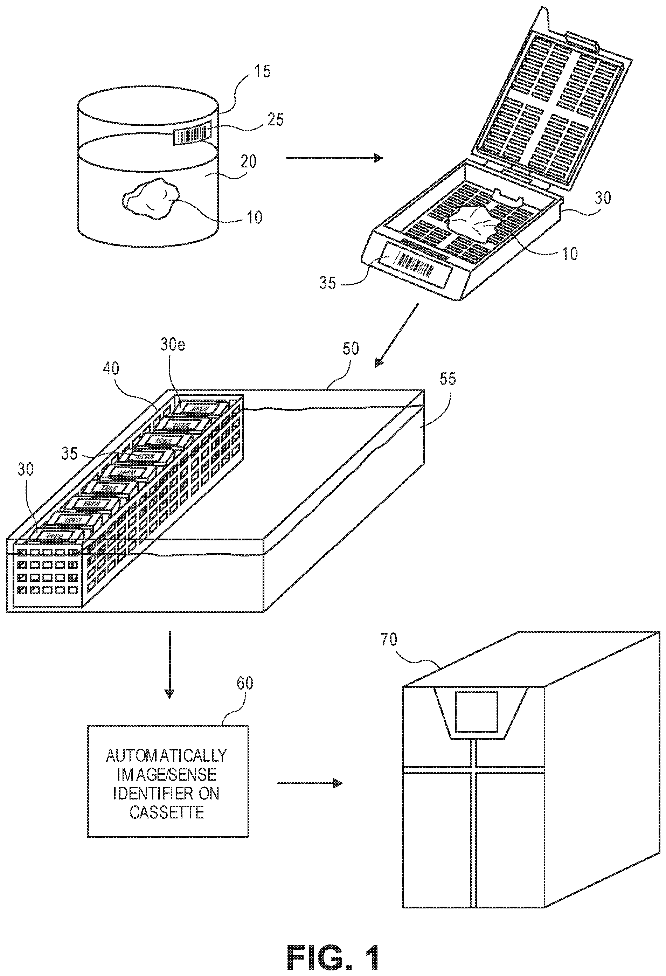

**Figure 1** illustratively shows a representative process flow of a tissue sample prior to tissue processing described above (e.g., prior to dehydration, clearing, paraffin infiltration and embedding). **Figure 1** shows tissue sample 10 that has been, for example, taken from a body (e.g., a human body) and placed in container 15 and immersed in fixative 20 such as formalin. In one example, tissue sample 10 may be obtained in a medical procedure by a physician that desires the tissue sample be evaluated for diagnostic or forensic purposes (e.g., evaluated for the presence of a disease). Container 15, in this embodiment, includes identifier 25 such as barcode that representatively contains or references information such as the name of the patient from where the tissue was extracted, physician identification information and optionally the desired processing protocol.

Prior to evaluation for diagnostic or forensic purposes, tissue sample 10 is subjected to tissue processing. To prepare for tissue processing, tissue sample 10 is transferred in whole or in part from container 15 into cassette 30, such as a plastic cassette (e.g., a sectionable cassette). Cassette 30 is generally a rectangular box having an interior volume defined by sidewalls and a base and having a retractable lid. A front sidewall or face of cassette 30 may be disposed at, for example, a 45-75 degree angle relative to the base with the other sidewalls at approximately a 90 degree angle relative to the base. Identifier 35 such as a barcode that contains or references information such as patient information, physician information and optionally processing protocol for the tissue sample is affixed by a technician to the front sidewall or face of cassette 30.

Once tissue sample 10 or a portion thereof is contained in cassette 30 (with lid closed), cassette 30 is placed in magazine 40, in one embodiment, along with other cassettes that are to be subjected to a similar tissue processing protocol. Representative magazines include Tissue-Tek AutoTEC^{®} magazines and Tissue-Tek Xpress^{®} magazines, where Tissue-Tek AutoTEC^{®} and Tissue-Tek Xpress^{®} are trademarks of Sakura Finetek USA, Inc. In another embodiment, magazine 40 may be a tissue cassette basket such as a cassette basket used in a Tissue-Tek VIP^{®} tissue processor, where Tissue-Tek VIP^{®} is a trademark of Sakura Finetek. As illustrated in **Figure 1****,** each cassette is placed in magazine 40 in an upright position so that the front sidewall or face of each cassette faces out the top of the magazine as viewed.

As noted above, in an embodiment, where cassettes such as cassette 30 are placed in a magazine according to a tissue processing protocol to be performed on each tissue sample (each tissue sample will receive the same tissue processing protocol), in one embodiment a reference cassette, such as an empty cassette (cassette 30e), may be included in the magazine. Cassette 30e includes an identifier on its front sidewall or face that specifically associates the magazine (magazine 40) with a particular tissue processing protocol and/or that contains information about the particular tissue processing protocol.

Referring to **Figure 1****,** magazine 40 is placed into a volume of JAR 50 that includes a fixative (fixative 55) in a volume therein to inhibit tissue samples in individual cassettes from drying out. In one embodiment, magazine 40 is placed into JAR 50 prior to loading cassettes into the magazine to ensure tissue samples in cassettes do not dry out and continue to be fixed or fixated. Magazine 40 can reside in JAR 50 either alone or with other magazines. JAR 50 may include features such as tabs that direct an alignment of a magazine in the JAR. In one embodiment, JAR 50 includes sidewalls and a base that define a volume. A top surface of JAR 50 may be exposed (no lid).

According to one embodiment, once the cassettes each containing a tissue sample are contained in a magazine in JAR 50, the identifier associated with each cassette (on a front sidewall or face) is automatically sensed by a device or apparatus that is operable to identify an identifier on an individual tissue cassette (block 60, **Figure 1**). The identifier on each cassette may be sensed while the cassette remains in a magazine in JAR 50. The sensing may be done individually by, for example, automatically moving a sensor over each cassette in a magazine or magazines in JAR 50 or sensed collectively by, for example, sensing (e.g., scanning, photographing) an image of multiple cassettes in a magazine (e.g., all the cassettes) or multiple magazines in JAR 50 (all the cassettes in a portion of JAR container 50 including an entire portion). Sensing identifiers associated with cassettes at this time identifies each tissue sample prior to tissue processing. Such feature offers a quality control function by providing a check that a cassette or cassettes in a magazine destined for a particular tissue processing protocol are intended for that protocol. Where a reference cassette is in a magazine including an identifier that associates the magazine with a particular tissue processing protocol (e.g., cassette 30e), such identifier information may be compared with identifier information of each of the other tissue cassettes in the particular magazine. If after the comparison, the identifiers on all the cassettes in the magazine share the same tissue processing protocol as the reference cassette, the tissue processing protocol is initiated on all the cassettes. If the identifier on one or more cassettes in the magazine does not share the same tissue processing protocol as the reference cassette, such one or more cassettes can be removed from the magazine before tissue processing. Sensing identifiers prior to tissue processing also offers a tracking function in the sense that a location of a tissue sample is known. Sensing identifiers prior to tissue processing also offers a routing function where the cassettes have been identified for a particular tissue processing protocol which may serve to alert a technician as to the protocol to perform on the cassettes. Following the sensing of an identifier for each cassette in a magazine 40, the magazine 40 may be placed in a tissue processor 70 such as Tissue-Tek VIP^{®} tissue processor.

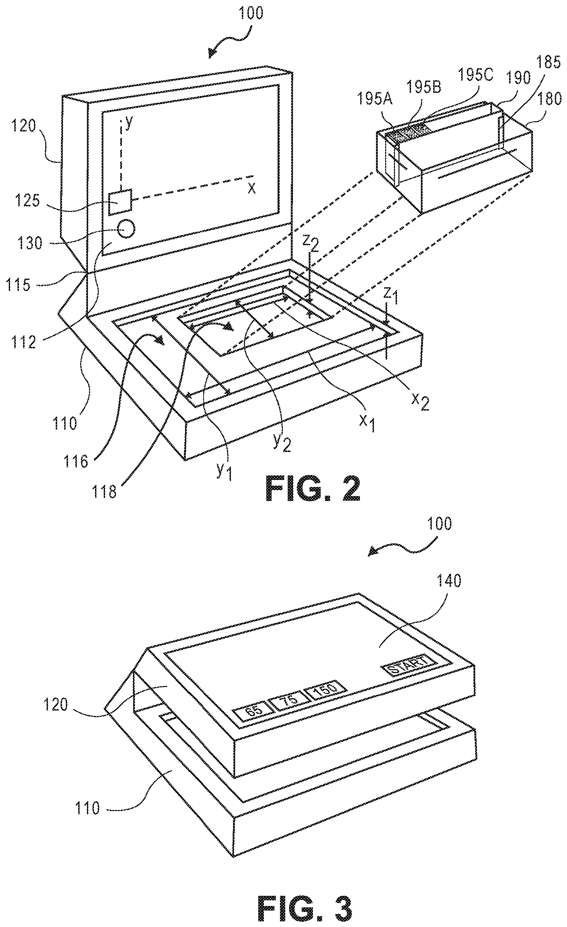

**Figure 2** shows a top side perspective view of an embodiment of an electronic device or apparatus that can sense, read, store and output printed information such as a barcode on a sample carrier. Device 100 is operable to be mounted on a table or other surface and have sample carriers such as cassettes be brought to the device. Device 100 includes base 110 and lid or cover 120 hingedly connected to base 110 through, for example, hinge 115. In one embodiment, the electronics for a barcode reader reside in lid 120. Such electronics representatively include a light source, lens and light sensor translating optical impulses into electrical ones. In another embodiment, a barcode scanner may be based on a light source and photodiode configuration wherein a photodiode(s) measures an intensity of a light reflected back from light source from the sample carrier. In another embodiment, the electronics for a barcode reader include a charge coupled device (CCD) reader or a camera and image processing techniques to decode a barcode and/or human readable text. A camera embodiment offers a benefit of not only being useful as a barcode reader but, when information on a barcode cannot be read by the barcode reader, the camera offers a picture of the barcode and possibly other readable information. Device 100 may therefore, in another embodiment, also include or alternatively include optical character recognition (OCR) processing capability. For purposes of the following discussion, a barcode reader will be described operable to sense barcodes on cassettes. Other sample carrier identification readers such as RFID and others are also contemplated.

**Figure 2** representatively shows light source 125 and sensor 130 within a body of lid 120, optionally behind glass cover 112. In one embodiment, the light source and sensor within a body of lid 120 are connected to and controlled by a Cartesian coordinate robot or linear robot and may be moved in x- and y-dimensions, as viewed, according to machine implemented (e.g., computer implemented) software instructions associated with or transmitted to device 100.

**Figure 2** shows container 180 that has a volume that is operable to contain a number of tissue cassettes or magazines of tissue cassettes therein. In one embodiment, container 180 is a JAR container that representatively is filled with a fixative and can contain multiple magazines of cassettes (e.g., an Tissue-Tek AutoTEC^{®} magazines and Tissue-Tek Xpress^{®} magazines) or a tissue cassette basket such as a cassette basket used in a Tissue-Tek VIP^{®} tissue processor. **Figure 2** representatively shows JAR container 180 including one magazine in a volume therein. Magazine 190 includes a number of cassettes (cassette 195A, cassette 195B, cassette 195C etc.). Each cassette, in one embodiment, has a barcode label printed on a front sidewall or face thereof facing outward from magazine 190 and a volume of JAR container 180 (upward as viewed). JAR container 180, in this embodiment, includes tabs 185 extending inward from opposing sidewalls that are operable to keep magazine 190 in place in the container and in a desired position and location.

Referring to **Figure 2****,** JAR container 180 is placed within base 110 of device 100. In one embodiment, base 110 includes one or more recesses formed therein to accommodate containers such as JAR container 180 of different sizes. The recesses are one form of orientation confiner or limiter that is intended to confine or limit the orientation of a container such as a JAR container 180 in base 110 to one orientation in which the container can be placed within base 110. Other confiners or limiters include an orientation used to locate each cassette in its carrier, and the carrier in the JAR container. In the illustrated embodiment, two different recesses are shown. Recess 116 occupies a majority of an area of base 110 and may be for a large basket or container (e.g., a JAR container) such as 150 basket JAR containers. Recess 116 is shown with x-dimension, x₁, and y-dimension, y₁ and z-dimension, z₁. Base 110 also includes small recess 118 within recess 116. Recess 118 may be suitable for small JAR containers such as a JAR container that may be operable to contain four magazines of e.g. twenty to thirty two cassettes each. Recess 118 is shown with x-dimension, x₂, y-dimension, y₂, and z dimension, z₂. Each recess serves to orient a container in base 110. In this example, the recesses are rectangular with an x-dimension greater than a respective y-dimension (x₂>y₂). A container (such as JAR container 180) will have similar dimensions so that it only fits within a recess (e.g., recess 118) with its longer dimension corresponding to the x-dimension of the recess.

Once a container such as container 180 is placed in a recess in base 110 of device 100, lid 120 of the device may be rotated over an exposed surface of base 110. **Figure 3** shows the device of **Figure 1** where lid 120 is rotated over base 110. In one embodiment, an area of lid 120 is operable to cover an area of base 110 so that the sensor 130 within lid 120 can be positioned as desired over all portions of a magazine or other cassette carrier located in a container (e.g., JAR container 180) placed into a recess (recess 116 or recess 118) in base 110. **Figure 3** shows lid 120 closed over base 110. Lid 120 has a first side through which sensor 130 has access therethrough to scan cassettes in base 110 and an opposite second side. According to **Figure 3****,** in one embodiment, an exposed or outer second side of lid 120 includes display 140. Display 140 may provide information to a user and allow interaction between the user and the device. Display 140, in one embodiment, is a touch display. A user can operate the device by logging into device 100 (e.g., by entering individual login information) using the touchscreen display (e.g., a keypad in display 140). A user can then press a start button to start the scan of the cassettes in a JAR container. Non-transitory program instructions within device 100 may direct a processor therein to automatically scan the recess regions of base 110 and read cassettes (scan barcodes) in magazines or baskets therein. A user may assist in the barcode scanning by instructing the device about the contents placed therein. For example, display 140 representatively includes touch pad identifiers (e.g., "65", "75", "150") that may be indicative of the container therein and for a user to press.

In one representative embodiment, device 100 has four modes of operation. A first mode of operation is that the device can scan barcodes and save them into a database within device 100. Secondly, device 100 can scan barcodes and save them into an on-board database and also send them to exterior systems such as laboratory information systems (LIS)/laboratory information management systems (LIMS)/Middleware computer software. A third mode of operation is similar to the second mode but device 100 can also request the LIS/LIMS/ Middleware computer software for tissue processing protocol information or can read the tissue processing protocol out of a barcode and analyze if there is any inconsistency related to tissue processing protocols between the cassettes. Finally, a fourth mode of operation allows device 100 to scan barcodes and save them into a database associated with the device and also to identify a color of a cassette and analyze if there is any inconsistency related to tissue processing protocols defined by a color of a cassette. The results of the different operations, in one embodiment, are displayed on display 140.

In an operation, the light source and barcode reader is moved by operation of the linear robot over each cassette in a container within base 110. In one embodiment, a basket or magazines has slots for each cassette. With a container positioned within a recess in base 110 (in a single permitted configuration to orient the slots of a magazine or baskets), the linear robot is programmed to position itself over each slot where a cassette may be present. When a cassette is present, a barcode on the cassette is scanned. Referring to **Figure 2** and **Figure 3****,** a first side of bottom of lid 120 contains the mechanics, light source and optics to read a barcode. When lid 120 is in a closed position over base 110, the light source and sensor 130 may be moved over each magazine or basket. In another embodiment, the program instructions associated with device include manual operation allowing a user to move the barcode scanner by commands introduced through a keypad on display 140.

The imaging device and systems described allows the sensing (e.g., reading) of barcodes of individual cassettes assembled together with other cassettes without removing a cassette from other cassettes in a magazine or basket. Also, the cassette may be disposed in a liquid such as a fixative at the time of barcode reading. Other benefits include:
1) The pre-loaded configurations of the device enable loading of different types of baskets and magazines into the device.
2) PC to provide UI, process images and communication to LIS/LIMS/middleware.
3) Touchscreen for receiving information from user and provide information to user.
4) Software: module to analyze the tissue processing protocol compatibility of cassettes, module to analyze the images and read out the barcode and or text, module to store all created information into an on-board database, module to report results (cassette barcode, position in basket/magazine, date and time, etc.) on screen and in file, module to communicate with LIS/ LIMS/middleware, module to set up and identify users (login), module to retrieve and collate information from cassettes stored into the database based on user queries/criteria.
5) Relatively fast barcode and fast reading camera to image the printed information on the writing surface of the cassette.
6) Barcode recognition of different types used in pathology laboratories (e.g., 2D datamatrix, 1D Code 129, Atzek).
7) True walkaway, error preventing process.
8) Automates the documentation used for tracking and tracing (e.g. into which tissue processor a cassette was loaded, for which tissue processing protocol, when, and by whom).
9) Automates tissue processing protocol verification process for cassettes.
10) Reporting of results on a device display and in file format (e.g., PDF) with the pictures of all cassette writing surfaces and location in the basket/magazine.
11) Reports can be created based on user defined queries/criteria.
12) LED illuminating the cassette without reflection for consistent image quality for all cassettes created by CCD type readers.
13) Reliable and fast automated reader of IDs of cassettes loaded into baskets or magazines before they are loaded into tissue processors.
14) Create documentation, verification and statistics on all cassettes loaded to device.
15) Minimal hands-on time (load JAR container, start process, unload JAR container).
16) Imaging device capable of reliably reading the barcode printed on an angled surface of cassettes in fixative.

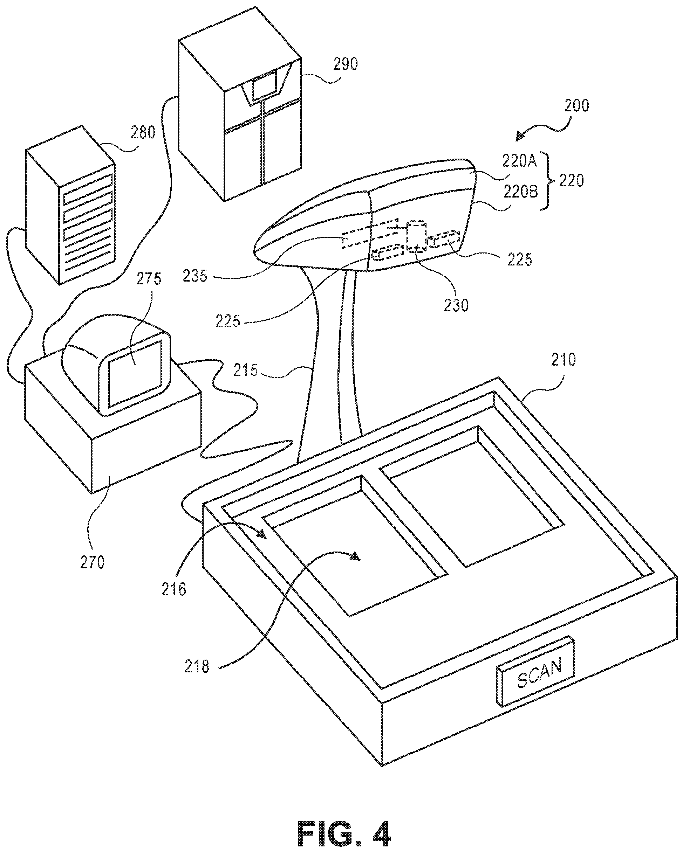

**Figure 4** shows a top perspective view of another embodiment of an electronic device or apparatus that can automatically sense, read, store and output printed information such as a barcode on a sample carrier. Device 200 is operable to be mounted on a table or other surface and have sample carriers such as cassettes be brought to the device. Device 200 includes40 210 and lid or cover 220 fixedly connected to base 210 through support 215. In one embodiment, lid 220 includes fixed portion 220A and rotating portion 220B. The electronics for a barcode scanner reside in portion 220B. Such electronics representatively include light source 225 (e.g., one or more light emitting diode (LED) structures (two shown)) lens 230 and light sensor 235 such as a charge-coupled device (CCD) linear image scanner. In one embodiment, the electronics for a barcode scanner are similar to the electronics in a Fujitsu Image Scanner ScanSnap SV600 available from Fujitsu Ltd. In another embodiment, electronics for light sensor 235 is a CCD or complementary metal oxide semiconductor (CMOS) camera operable to digitally capture a three-dimensional image at a high resolution (e.g., 1600x1200 or higher). In another embodiment, electronics in device 200 include both a scanner to capture a two-dimensional image and a camera to capture a three-dimensional image.

In one embodiment, base 210 of device 200 includes one or more recesses formed therein to accommodate containers such JAR containers of different sizes similar to base 110 described above with reference to **Figures 2** and **3****.** The recesses are one form of orientation confiner or limiter that is intended to confine or limit the orientation of a container such as a JAR container in base 210 to one orientation in which the container can be placed within base 210. In the illustrated embodiment, three different recesses are shown. Recess 216 occupies a majority of an area of base 210 and may be for a large basket or container. Base 210 also includes two smaller recesses 218 within recess 216. Each recess 218 may be suitable for small JAR containers such as a JAR container that may be operable to contain four magazines of e.g., 20 to 32 cassettes each. In another embodiment, base 210 provides support for the lid (through arm 215) but does not include a recess for a container or containers.

In one embodiment, device 200 shown in **Figure 4** is connected to processor 270. Processor 270 in one embodiment contains information about a tissue processing protocol for cassettes in a container. In one embodiment, device 200 is operable to scan images of identifiers (e.g., barcodes) on cassettes in a container, convert the scanned images into electronic information (signals) and provide the electronic information to processor 270. Processor 270 contains non-transitory program instructions operable to obtain the electronic information of the identifiers; to compare that information to information previously entered or retrieved from server 285 that may be, for example, a laboratory information system (LIS) about a desired tissue processing protocol; and to report and record the comparison including to report an error to a technician about a tissue sample before a tissue processing protocol is initiated. For example, processor 270, in one embodiment, includes display 275 and program instructions in the processor are operable to cause processor 270 to display a signal such as an alarm or error code on display 275. Processor 270 as a form of Middleware provides a verification process that a tissue sample in a magazine in a container is intended for a particular tissue processing protocol for other samples in the magazine or container. In another embodiment, a magazine in a container may contain a reference such as, as described above, a reference cassette with an identifier indicative of an intended tissue processing protocol. Processor 270 contains non-transitory program instructions operable to obtain the electronic information of the identifiers including an identifier on the reference (e.g., identifier on the reference cassette) and compare the electronic information of the other cassettes in the container with that of the reference to verify the tissue processing protocol of the other samples in the container. In a further embodiment, processor 270 may use information related to a color of a cassette (e.g., reference or reference cassette is color) to verify a tissue processing protocol of the tissue samples. Device 200 is operable to provide an image of an identifier on each cassette in a container (e.g., a two dimensional image). That image information captured, in one embodiment, also includes the color of the front side or face of a cassette. In a still further embodiment, processor 270 contains non-transitory program instructions operable to obtain the electronic information of the identifiers from device 200 and then inform a technician of a desired tissue processing protocol of the tissue samples in a scanned container based on the obtained information from the identifiers. Processor 270, in one embodiment, retrieves tissue processing information from server 285 and informs a technician of the tissue processing information (such as through display 275) following the scanning of identifiers on cassettes in container.

In a still further embodiment, a tissue processor is programmed to execute a tissue processing protocol based on the scanned identifiers in a particular magazine or a reference cassette or color of cassettes in a particular magazine. In one embodiment, processor 270 is connected to tissue processor 290 to effect the programming of tissue processor 290. In a still further embodiment, a technician can scan a reference cassette and the selected processing protocol of a tissue processor to confirm a match prior to executing the protocol.

In one embodiment, display 275 allows interaction between a user (e.g., a technician) and device 200. Such interaction includes, but is not limited to, initiating a scanning of a container on base 210 or a repeat of a scanning of a container.

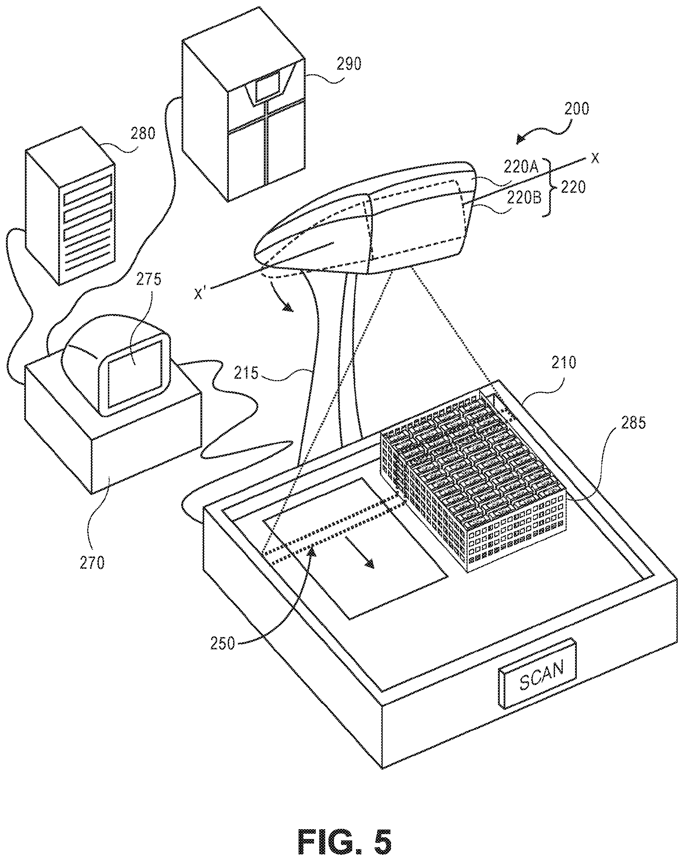

**Figure 5** shows the device of **Figure 4** including container 280 containing a fixative and four magazines filled with cassettes disposed in recess 218 of base 210 of the device. Each magazine in container 280 may contain tissue samples intended for different tissue processing protocols. In other words, one magazine in container 280 may contain cassettes intended to be processed according to a first tissue processing protocol and a second magazine containing cassettes intended to be processed according to a second tissue processing protocol. It is appreciated that another container may be placed, representatively in the second recess 218 and both containers scanned simultaneously. **Figure 5** shows a scanning operation of device 200. One device 200 is activated (via an on/off switch), a technician may push a "scan" button on the device or initiate a scan through processor 270. In this embodiment, in a scan operation, portion 220B of lid 220 rotates about axis x-x' to allow light source 225 and lens 230 to advance row by row of cassettes from one side of base 210 to an opposite side. In this manner, device 200 scans/captures an image of identifiers on four cassettes at a time (one cassette in each of four magazines arranged side by side). **Figure 5** shows beam 250 across base 210 indicative of a scan. The rotation of lid 220 about axis x-x¹ allows beam 250 to advance on a surface of base 210 from one side of the base 210 closest to support 215 to an opposite side. In one embodiment, device 200 scans an image of the identifiers and saves the image into a database within the device. When all rows of cassettes are scanned, a processor associated with device 200 constructs a single image of all the cassettes showing their respective identifiers. The image is then converted into an electronic information (signals) and then provided, in one embodiment, to processor 270. Processor 270 contains non-transitory program instructions to receive the electronic information and confirm that the electronic information is indicative of identifier information for each cassette in the container. An image of all the identifiers, in one embodiment, is optionally displayed on display 275. In an embodiment where a scan is unable to capture an identifier on a cassette, the program instructions associated with processor 270 are operable to alert a technician through, for example, an alert on display 275. Such an alert allows a technician to scan the missing identifier manually or enter the identifier manually into processor 270.

The imaging device and systems described allows the sensing (e.g., imaging) of identifiers barcodes of individual cassettes assembled together with other cassettes without removing a cassette from other cassettes in a magazine or basket. Also, the cassette may be disposed in a liquid such as a fixative at the time of barcode sensing.

In the description above, for the purposes of explanation, numerous specific details have been set forth in order to provide a thorough understanding of the embodiments. It will be apparent however, to one skilled in the art, that one or more other embodiments may be practiced without some of these specific details. The particular embodiments described are not provided to limit the invention but to illustrate it. The scope of the invention is not to be determined by the specific examples provided above but only by the claims below. In other instances, well-known structures, devices, and operations have been shown in block diagram form or without detail in order to avoid obscuring the understanding of the description. Where considered appropriate, reference numerals or terminal portions of reference numerals have been repeated among the figures to indicate corresponding or analogous elements, which may optionally have similar characteristics.

It should also be appreciated that reference throughout this specification to "one embodiment", "an embodiment", "one or more embodiments", or "different embodiments", for example, means that a particular feature may be included in the practice of the invention. Similarly, it should be appreciated that in the description various features are sometimes grouped together in a single embodiment, figure, or description thereof for the purpose of streamlining the disclosure and aiding in the understanding of various inventive aspects. This method of disclosure, however, is not to be interpreted as reflecting an intention that the invention requires more features than are expressly recited in each claim. Rather, as the following claims reflect, inventive aspects may lie in less than all features of a single disclosed embodiment. Thus, the claims following the Detailed Description are hereby expressly incorporated into this Detailed Description, with each claim standing on its own as a separate embodiment of the invention.

## Claims

1. An apparatus (200) to identify an identifier on a tissue cassette (30) in an assembly of a plurality of tissue cassettes (180; 280) comprising:
a light source (225) operable to illuminate the plurality of tissue cassettes in the assembly (280), the assembly (280) comprising sidewalls and a base that define a volume and an exposed top surface, the assembly capable of containing a plurality of tissue cassettes together in an upright position in rows and columns so that a front sidewall of each of the plurality of tissue cassettes faces outward from the top surface of the assembly; and
a sensor (235) operable to automatically capture an identifier on an individual tissue cassette (30) or image data of the plurality of tissue cassettes (30) in the assembly (280), wherein the sensor (235) is operable to capture image data of the plurality of tissue cassettes, a converter to convert the image data into an electronic signal,
the apparatus comprising a processor comprising non-transitory program instructions, the apparatus includes a base (210) to contain the assembly of a plurality of tissue cassettes (280) and a lid (220) coupled to the base (210), the lid (220) comprising the light source (225), and the sensor (235), **characterized by**:
the lid being rotatable about an axis x-x' over the base and the non-transitory program instructions being configured, when executed on the processor, to (i) direct a rotational movement of the sensor (230) about the axis x-x' to allow a capture of an identifier on an individual tissue cassette (30) positioned anywhere in the assembly of a plurality of tissue cassettes (280), (ii) cause the sensor to automatically capture an identifier on each individual tissue cassette while each individual tissue cassette is contained in the assembly of a plurality of tissue cassettes (280) and, (iii) after capturing an identifier of each individual tissue cassette in the plurality of tissue cassettes (280), construct a single image of the plurality of tissue cassettes contained in the assembly showing their respective identifiers.

2. The apparatus (200) of claim 1, wherein the base (210) comprises an orientation designation for a container operable to contain the assembly of the plurality of tissue cassettes (280).

3. The apparatus (100; 200) of claim 2, wherein the sensor (235) comprises a barcode reader.

4. The apparatus (200) of claim 2, wherein the base (210) comprises at least one recess (216, 218) formed therein, the at least one recess (216, 218) comprising dimensions to contain a container in the base at the orientation designation, wherein the container is operable to contain the assembly of the plurality of tissue cassettes (180; 280).

5. The apparatus (200) of claim 4, wherein the at least one recess (216, 218) comprises at least two recesses (216, 218).

6. The apparatus (200) of claim 5, wherein one of the at least two recesses ( 216, 218) is operable to contain a first container having first dimensions and the other of the at least two recesses (216, 218) is operable to contain a second container having second dimensions different than the first dimensions.

7. The apparatus (200) of claim 1, wherein the sensor (235) is operable to sense an image of the plurality of tissue cassettes (280) in the assembly.

8. The apparatus (200) of claim 1, wherein the apparatus (200) comprises at least one of the following modes of operation:
save the captured light and the constructed image in a database within the apparatus (200);
send the captured light and the constructed image to an exterior system;
request processing protocol information from an exterior system and analyze whether there is any inconsistency between the information associated with the captured light and the constructed image and the processing protocol; or
identify a color of the individual tissue cassette (280) based on the constructed image and
analyze whether there is any inconsistency between the information associated with the identifier and a processing protocol defined by a color of the individual tissue cassette (280).

9. A system comprising:
the apparatus (200) of any of claims 1-8; and
a processor (270) coupled to the apparatus (200), the processor comprising non-transitory program instructions operable
i) to obtain electronic information of the identifier;
ii) to compare that information to information previously entered or retrieved information about a desired tissue processing protocol; and
iii) to report the comparison.

10. The system of claim 9, wherein the retrieved information comprises information from a referenced identifier in the assembly.

11. The system of claim 9, wherein the retrieved information comprises information regarding a color of a tissue cassette (280).

12. A method for using the apparatus of any of claims 1-9 comprising:
sensing identifiers on tissue cassettes (280) each containing a tissue sample, the sensing occurring while the tissue cassettes (280) are in an assembly comprising a plurality of tissue cassettes (280).

13. The method of claim 12, wherein sensing comprises capturing image data of a plurality of tissue cassettes (280) in the assembly showing their respective identifiers.

14. The method of claim 12, wherein sensing comprises capturing an identifier on an individual tissue cassette (280) in the assembly.

## Patentansprüche

1. Vorrichtung (200) zum Identifizieren einer Kennung auf einer Gewebeeinbettkassette (30) in einer Gruppe aus einer Mehrzahl von Gewebeeinbettkassetten (180; 280), umfassend:
eine Lichtquelle (225), die betriebsfähig ist, die Mehrzahl von Gewebeeinbettkassetten in der Gruppe (280) zu beleuchten, wobei die Gruppe (280) Seitenwände und einen Boden umfasst, die einen Innenraum und eine freiliegende obere Oberfläche begrenzen, und wobei die Gruppe in der Lage ist, eine Mehrzahl von Gewebeeinbettkassetten gemeinsam in einer aufrechten Stellung in Reihen und Spalten aufzunehmen, sodass eine vordere Seitenwand jede der Mehrzahl von Gewebeeinbettkassetten von der oberen Oberfläche der Gruppe nach außen zeigt; und
einen Sensor (235), der betriebsfähig ist, automatisch eine Kennung auf einer einzelnen Gewebeeinbettkassette (30) oder Bilddaten der Mehrzahl von Gewebeeinbettkassetten (30) in der Gruppe (280) zu erfassen, wobei der Sensor (235) dazu ausgelegt ist, Bilddaten der Mehrzahl von Gewebeeinbettkassetten zu erfassen, einen Wandler zum Umwandeln der Bilddaten in ein elektronisches Signal,
wobei die Vorrichtung einen Prozessor umfasst, der nichtflüchtige Programmbefehle umfasst,
wobei die Vorrichtung eine Basis (210) zur Aufnahme der Gruppe aus einer Mehrzahl von Gewebeeinbettkassetten (280) und einen mit der Basis (210) verbundenen Deckel (220) umfasst, wobei der Deckel (220) die Lichtquelle (225) und den Sensor (235) umfasst, **gekennzeichnet dadurch, dass**:
der Deckel um eine Achse x-x' über der Basis drehbar ist
und die nichtflüchtigen Programmbefehle so konfiguriert sind, dass sie bei Ausführung auf dem Prozessor (i) eine Drehbewegung des Sensors (230) um die Achse x-x' steuern, um die Erfassung einer Kennung auf einer einzelnen Gewebeeinbettkassette (30) zu ermöglichen, die an einer beliebigen Stelle in der Gruppe aus einer Mehrzahl von Gewebeeinbettkassetten (280) positioniert ist, (ii) den Sensor dazu zu veranlassen, automatisch eine Kennung auf jeder einzelnen Gewebeeinbettkassette zu erfassen, während sich jede einzelne Gewebeeinbettkassette in der Gruppe aus einer Mehrzahl von Gewebeeinbettkassetten (280) befindet, und (iii) nach dem Erfassen einer Kennung jeder einzelnen Gewebeeinbettkassette aus der Mehrzahl von Gewebeeinbettkassetten (280) ein einziges Bild der in der Gruppe enthaltenen Mehrzahl von Gewebeeinbettkassetten zu erstellen, das deren jeweilige Kennungen zeigt.

2. Vorrichtung (200) nach Anspruch 1, wobei die Basis (210) eine Ausrichtungsmarkierung für einen Behälter aufweist, der betriebsfähig ist, die Gruppe der Mehrzahl von Gewebeeinbettkassetten (280) aufzunehmen.

3. Vorrichtung (100; 200) nach Anspruch 2, wobei der Sensor (235) einen Barcode-Leser umfasst.

4. Vorrichtung (200) nach Anspruch 2, wobei die Basis (210) mindestens eine darin ausgebildete Aussparung (216, 218) umfasst, wobei die mindestens eine Aussparung (216, 218) Abmessungen umfasst, um einen Behälter in der Basis an der Ausrichtungsmarkierung aufzunehmen, wobei der Behälter betriebsfähig ist, die Gruppe der mehreren Gewebeeinbettkassetten (180; 280) aufzunehmen.

5. Vorrichtung (200) nach Anspruch 4, wobei die mindestens eine Aussparung (216, 218) mindestens zwei Aussparungen (216, 218) umfasst.

6. Vorrichtung (200) nach Anspruch 5, wobei eine der mindestens zwei Aussparungen (216, 218) betriebsfähig ist, einen ersten Behälter mit ersten Abmessungen aufzunehmen, und die andere der mindestens zwei Aussparungen (216, 218) betriebsfähig ist, einen zweiten Behälter mit zweiten Abmessungen aufzunehmen, die sich von den ersten Abmessungen unterscheiden.

7. Vorrichtung (200) nach Anspruch 1, wobei der Sensor (235) betriebsfähig ist, ein Bild der Mehrzahl von Gewebeeinbettkassetten (280) in der Gruppe zu erfassen.

8. Vorrichtung (200) nach Anspruch 1, wobei die Vorrichtung (200) mindestens einen der folgenden Betriebsmodi umfasst:
Speichern des erfassten Lichts und des rekonstruierten Bildes in einer Datenbank innerhalb der Vorrichtung (200); Senden des erfassten Lichts und des rekonstruierten Bildes an ein externes System;
Anfordern von Informationen zum Verarbeitungsprotokoll von einem externen System und Analysieren, ob eine Unstimmigkeit zwischen den mit dem erfassten Licht und dem rekonstruierten Bild verbundenen Informationen und dem Verarbeitungsprotokoll vorliegt; oder
Identifizieren einer Farbe der einzelnen Gewebeeinbettkassette (280) basierend auf dem rekonstruierten Bild und Analysieren, ob eine Unstimmigkeit zwischen den mit der Kennung verbundenen Informationen und einem durch eine Farbe der einzelnen Gewebeeinbettkassette (280) definierten Verarbeitungsprotokoll vorliegt.

9. System, umfassend:
die Vorrichtung (200) nach einem der Ansprüche 1-8; und
einen mit der Vorrichtung (200) gekoppelten Prozessor (270), wobei der Prozessor nichtflüchtige Programmbefehle umfasst, die betriebsfähig sind,
i) um elektronische Informationen der Kennung zu erhalten;
ii) um diese Informationen mit zuvor eingegebenen oder abgerufenen Informationen über ein gewünschtes Gewebeverarbeitungsprotokoll zu vergleichen; und
iii) um den Vergleich zu melden.

10. System nach Anspruch 9, wobei die abgerufenen Informationen Informationen aus einer referenzierten Kennung in der Gruppe umfassen.

11. System nach Anspruch 9, wobei die abgerufenen Informationen Informationen bezüglich einer Farbe einer Gewebeeinbettkassette (280) umfassen.

12. Verfahren zum Verwenden der Vorrichtung nach einem der Ansprüche 1 bis 9, umfassend:
Erfassen von Kennungen an Gewebeeinbettkassetten (280), die jeweils eine Gewebeprobe enthalten, wobei das Erfassen stattfindet, während sich die Gewebeeinbettkassetten (280) in einer Gruppe befinden, die eine Mehrzahl von Gewebeeinbettkassetten (280) umfasst.

13. Verfahren nach Anspruch 12, wobei das Erfassen das Aufnehmen von Bilddaten einer Mehrzahl von Gewebeeinbettkassetten (280) in der Gruppe umfasst, die ihre jeweiligen Kennungen zeigen.

14. Verfahren nach Anspruch 12, wobei das Erfassen das Aufnehmen einer Kennung auf einer einzelnen Gewebeeinbettkassette (280) in der Gruppe umfasst.

## Revendications

1. Appareil (200) destiné à identifier un identifiant sur une cassette de tissu (30) dans un ensemble composé d'une pluralité de cassettes de tissu (180 ; 280) comprenant :
une source lumineuse (225) pouvant éclairer la pluralité de cassettes de tissu dans l'ensemble (280), l'ensemble (280) comprenant des parois latérales et une base qui définissent un volume et une surface supérieure exposée, l'ensemble pouvant contenir une pluralité de cassettes de tissu ensemble en position verticale, disposées en rangées et en colonnes, de telle sorte qu'une paroi latérale avant de chacune de la pluralité de cassettes de tissu soit tournée vers l'extérieur depuis la surface supérieure de l'ensemble ; et
un capteur (235) pouvant capturer automatiquement un identifiant sur une cassette de tissu individuelle (30) ou des données d'image de la pluralité de cassettes de tissu (30) dans l'ensemble (280), dans lequel le capteur (235) peut capturer des données d'image de la pluralité de cassettes de tissu, un convertisseur pour convertir les données d'image en un signal électronique,
l'appareil comprenant un processeur comprenant des instructions de programme non transitoires,
l'appareil comprend une base (210) destinée à contenir l'ensemble composé d'une pluralité de cassettes de tissu (280) et un couvercle (220) couplé à la base (210), le couvercle (220) comprenant la source lumineuse (225) et le capteur (235), **caractérisé en ce que** :
le couvercle pouvant tourner autour d'un axe x-x' au-dessus de la base
et les instructions de programme non transitoires étant configurées, lorsqu'elles sont exécutées sur le processeur, pour (i) commander un mouvement de rotation du capteur (230) autour de l'axe x-x' afin de permettre la capture d'un identifiant sur une cassette de tissu individuelle (30) positionnée n'importe où dans l'ensemble composé d'une pluralité de cassettes de tissu (280), (ii) amener le capteur à capturer automatiquement un identifiant sur chaque cassette de tissu individuelle tant que chaque cassette de tissu individuelle est contenue dans l'ensemble composé d'une pluralité de cassettes de tissu (280) et, (iii) après avoir capturé un identifiant de chaque cassette de tissu individuelle parmi la pluralité de cassettes de tissu (280), construire une image unique de la pluralité de cassettes de tissu contenues dans l'ensemble, montrant leurs identifiants respectifs.

2. Appareil (200) selon la revendication 1, dans lequel la base (210) comprend une indication d'orientation pour un conteneur pouvant contenir l'ensemble composé de la pluralité de cassettes de tissu (280).

3. Appareil (100 ; 200) selon la revendication 2, dans lequel le capteur (235) comprend un lecteur de codes-barres.

4. Appareil (200) selon la revendication 2, dans lequel la base (210) comprend au moins un évidement (216, 218) formé dans celle-ci, l'au moins un évidement (216, 218) comprenant des dimensions destinées à contenir un récipient dans la base au niveau de l'indication d'orientation, le récipient pouvant contenir l'ensemble composé de la pluralité de cassettes de tissu (180 ; 280).

5. Appareil (200) selon la revendication 4, dans lequel l'au moins un évidement (216, 218) comprend au moins deux évidements (216, 218).

6. Appareil (200) selon la revendication 5, dans lequel l'un des au moins deux évidements (216, 218) peut recevoir un premier récipient présentant des premières dimensions et l'autre des au moins deux évidements (216, 218) peut recevoir un second récipient présentant des secondes dimensions différentes des premières dimensions.

7. Appareil (200) selon la revendication 1, dans lequel le capteur (235) peut détecter une image de la pluralité de cassettes de tissu (280) présentes dans l'ensemble.

8. Appareil (200) selon la revendication 1, dans lequel l'appareil (200) comprend au moins l'un des modes de fonctionnement suivants :
enregistrer la lumière capturée et l'image reconstruite dans une base de données au sein de l'appareil (200) ; envoyer la lumière capturée et l'image reconstruite à un système externe ;
demander des informations relatives au protocole de traitement à un système externe et analyser s'il existe une incohérence entre les informations associées à la lumière capturée, à l'image reconstruite et le protocole de traitement ; ou
identifier une couleur de la cassette de tissu individuelle (280) sur la base de l'image reconstruite et analyser s'il existe une incohérence entre les informations associées à l'identifiant et un protocole de traitement défini par une couleur de la cassette de tissu individuelle (280).

9. Système comprenant :
l'appareil (200) selon l'une quelconque des revendications 1 à 8 ; et
un processeur (270) couplé à l'appareil (200), le processeur comprenant des instructions de programme non transitoires pouvant être exécutées
i) pour obtenir des informations électroniques relatives à l'identifiant ;
ii) pour comparer ces informations à des informations précédemment saisies ou récupérées concernant un protocole de traitement de tissu souhaité ; et
iii) pour rendre compte de la comparaison.

10. Système selon la revendication 9, dans lequel les informations récupérées comprennent des informations provenant d'un identifiant référencé dans l'ensemble.

11. Système selon la revendication 9, dans lequel les informations récupérées comprennent des informations concernant la couleur d'une cassette de tissu (280).

12. Procédé d'utilisation de l'appareil selon l'une quelconque des revendications 1 à 9, comprenant :
la détection d'identifiants sur des cassettes de tissu (280) contenant chacune un échantillon de tissu, la détection ayant lieu alors que les cassettes de tissu (280) se trouvent dans un ensemble comprenant une pluralité de cassettes de tissu (280).

13. Procédé selon la revendication 12, dans lequel la détection comprend la capture de données d'image d'une pluralité de cassettes de tissu (280) dans l'ensemble, montrant leurs identifiants respectifs.

14. Procédé selon la revendication 12, dans lequel la détection comprend la capture d'un identifiant sur une cassette de tissu (280) individuelle dans l'ensemble.
